# EUROPEAN PATENT SPECIFICATION

(11) **EP 2 712 590 B1**
(45) Date of publication and mention of the grant of the patent: **08.04.2015**
(21) Application number: 13186427.4
(22) Date of filing: 27.09.2013
(51) Int. Cl.: A61F 2/46

(54) **Impactor for securing an implant to a bone surface**
Einschlaggerät zur Fixierung eines Implantats an einer Knochenoberfläche
Impacteur permettant de fixer un implant à une surface osseuse

(30) Priority: 28.09.2012 US 201213629943
(43) Date of publication of application: 02.04.2014
(73) Proprietor: DePuy (Ireland), Ringaskiddy (IE)
(72) Inventor: Wagner, Christel M, Warsaw, IN Indiana 46581 (US); Leestma, Scott M, Warsaw, IN Indiana 46581 (US)
(74) Representative: Belcher, Simon James

(56) References cited:
- EP-A1- 0 327 509
- EP-A1- 1 698 306
- EP-A2- 1 666 007
- US-A1- 2012 123 429

## Description

The present invention relates to a surgical instrument and, more particularly, to an impactor for use in securing an orthopaedic implant to a bone surface.

During orthopaedic arthroplasty procedures, impactors may be used to drive implant components into the healthy bone that remains. The impactors include a first end that applies force to an implant component and a second, opposite or free end. After resection/preparation of the bone surface, cement or a similar material may be applied to the prepared bone surface and the implant component is attached to the prepared bone surface. Thereafter, the impactor is situated adjacent the implant component and a tool, such as a hammer or mallet, is used to hit the free end of the impactor, thereby applying force to the implant component via the first end of the impactor and driving the implant component into the resected bone surface. The free end of the impactor may be hit multiple times to fully seat the implant component.

EP-A-1698306 discloses a tool for introducing an acetabular cup which has a head at one end. The head has a base plate and a central element for holding the cup. Pins are provided around the periphery of the base plate which are received in bores in the rim of the cup when the cup is in position on the head, with the rim of the cup in contact with the base plate.

US-A-2012/0123429 discloses an impactor surgical instrument for implanting femoral and tibial components of a knee prosthesis. The tibial component has a planar upper face and this face is contacted by three flat co-planar formations on the impactor instrument. The impactor instrument has a formation on one side which can be fitted into the gap between the condylar portions of the femoral component during implantation of the femoral component, for the purpose of applying an impaction force to the femoral component. The formation does not extend as far along the impactor instrument as the surfaces which contact the planar upper face of the tibial component.

The present invention provides an assembly for use in securing an orthopaedic implant within a surface of a patient's bone, as defined in claim 1.

Optionally, the projection is parallel to a longitudinal axis of the head and extends from an edge of the impaction surface.

Optionally, the head includes a cavity adapted to accept a projection extending outwardly from an implant.

Optionally, the head includes impaction surfaces on opposing sides of the cavity that are adapted to contact medial and lateral bearing surfaces or a perimeter of a surface of the tibial component, the cavity is adapted to accept a spine of the tibial component, and the anti-rotation projection extends into a groove formed by a concave posterior edge of the tibial component.

Optionally, the impaction surface is formed on opposing sides of the cavity and is generally planar.

Optionally, the impaction surface is formed on opposing sides of the cavity and includes convex surfaces that are adapted to conform to concave bearings surfaces of the implant.

Optionally, the shaft includes a connector that allows for attachment of different end pieces.

Optionally, the connector is formed by a lever arm that operates a latch finger having a latch at an end thereof that cooperates with a projection in each end piece to retain the end piece on the shaft.

Optionally, the impactor head is integrally and non-removably attached to the shaft.

The invention is described below by way of example with reference to the accompanying drawings, in which:
FIG. 1 is a perspective view of an impactor head attached to an end of a tool, in which the impactor head is used to aid in driving a tibial component into the tibia of a patient.
FIG. 2 is an exploded perspective view of the tool and impactor head of FIG. 1.
FIG. 3 is a bottom perspective view of an end of the impactor head shown in FIGS. 1 and 2 and depicting an anti-rotation feature and a cavity within the impactor head.
FIG. 4 is a perspective view of the tool of FIGS. 1 and 2 with the impactor head of FIGS. 1 to 3 attached thereto and depicting the tool and the impactor head as they are guided toward an implant component.
FIG. 5 is a perspective view of the tool and the impactor head of FIG. 4 after the tool has been guided toward the implant component and the impactor head is in contact with the implant component.
FIG. 6 is a cross-sectional view taken generally along the lines 6-6 of FIG. 5 and depicting a spine of the implant component within the cavity and further depicting the anti-rotation feature of the impactor head disposed within a groove formed by a concave surface on a posterior side of the implant component.

Terms representing anatomical references, such as anterior, posterior, medial, lateral, superior and inferior, may be used throughout this document to refer to both the orthopaedic implants described herein and a patient's natural anatomy. Such terms have well-understood meanings in both the study of anatomy and the field of orthopaedics. Use of such anatomical reference terms in this document is intended to be consistent with their well-understood meanings unless noted otherwise.

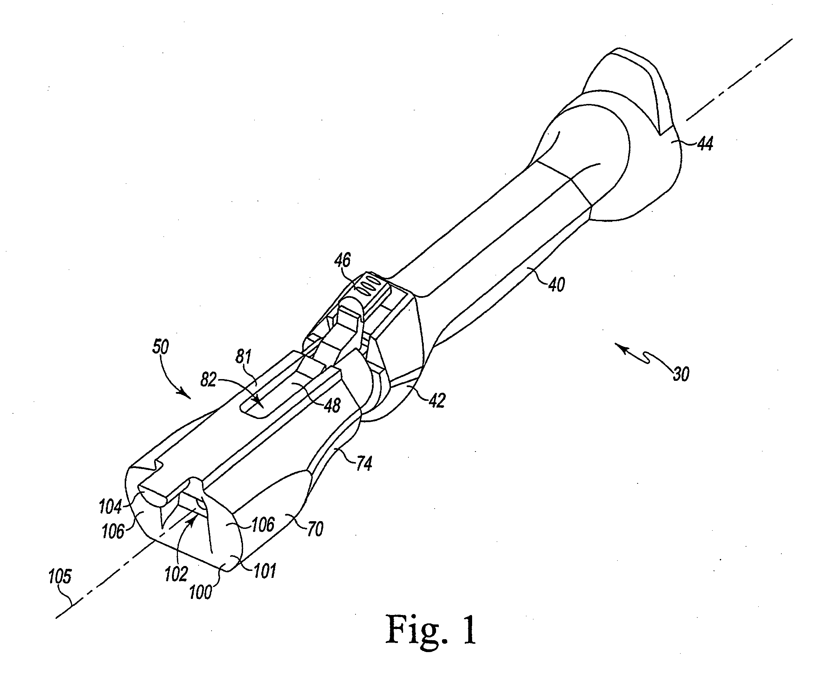

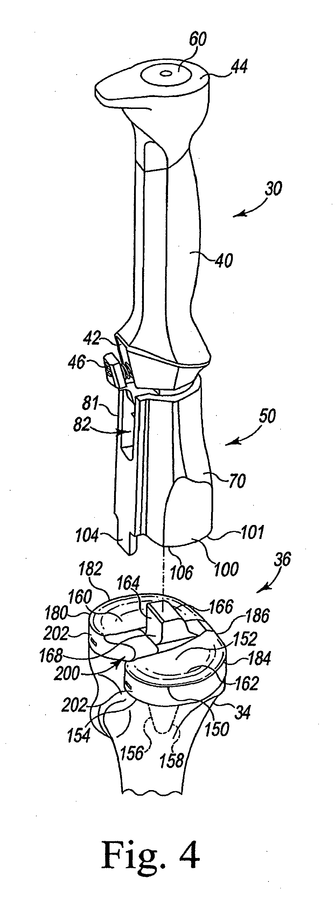

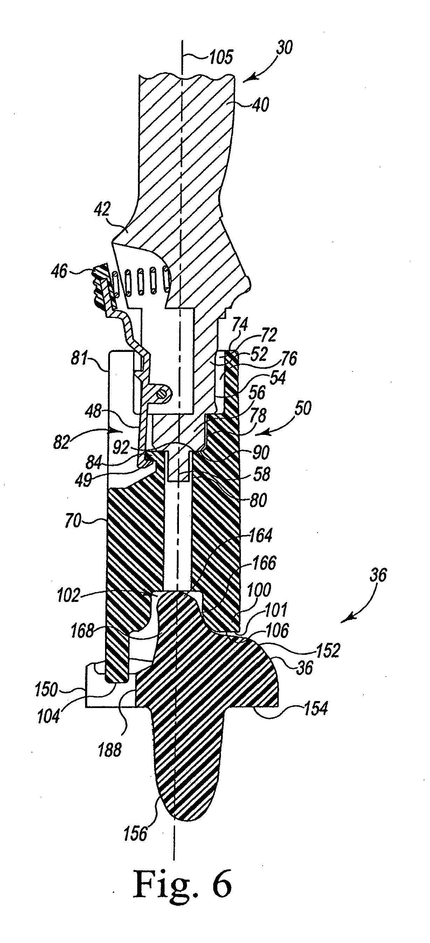

Referring to the drawings, FIGS. 1 to 3 show a tool 30 for driving an implant component (as shown in FIGS. 4 to 6) into a bone surface 34. The implant component is in the form of a tibial component 36 forming a portion of a posterior stabilized knee orthopaedic prosthesis in combination with a femoral component (not shown).

The tibial component 36 is illustratively formed from a polymeric material such as ultra-high molecular weight polyethylene (UHMWPE), but may be formed from other materials, such as a ceramic material, a metallic material, a bio-engineered material, or the like. The tibial component 36 is configured to be secured to the bone surface 34 in the form of a surgically-prepared proximal end of a patient's tibia and the femoral component is configured to articulate with the tibial component 36.

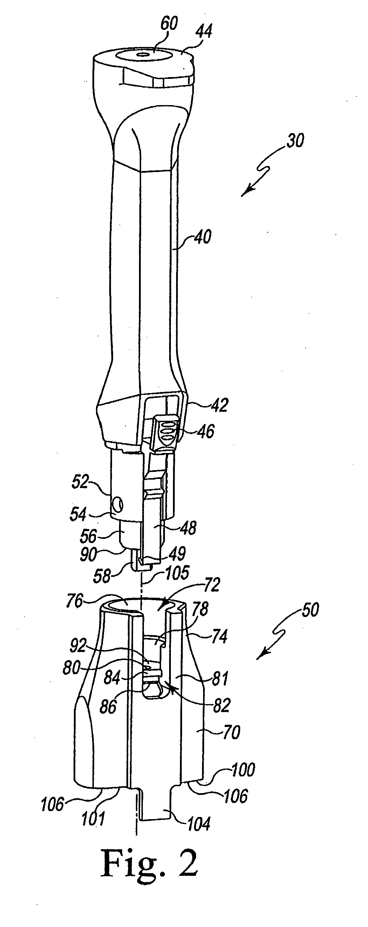

As best seen in FIGS. 1 and 2, the tool 30 includes a central shaft 40 having an attachment end 42 and a free end 44. The attachment end 42 includes a universal quick-release connector having a lever arm 46 that operates a latch finger 48. A latch 49 is disposed at an end of the latch finger 48 to easily attach and detach various instrument end pieces to the tool 30, for example, an impactor head 50. The latch finger 48 is biassed to a closed position such that inward movement of the lever arm 46 opens the latch 49. The attachment end 42 further includes a central projection 52 formed of a plurality of concentric cylinders 54, 56, 58 each extending from the next and each having a smaller diameter than the cylinder 54, 56, 58 from which it extends, with the cylinder 58 having a smallest diameter and the cylinder 54 having a largest diameter.

While the tool 30 is described herein as being connected to an impactor head 50, the universal connector of the tool 30 may be connected to any instrument end piece including, but not limited to, punches, reamers, inserters, extractors, impactors, or any other instruments in which use of a hand piece or tool for controlling the instrument end piece is desired. During a particular surgery, the tool 30 may be utilized with a plurality of different end pieces to minimize the number of tools necessary in an operating room. Optionally, the impactor head 50 may be integral with and non-removably attached to the tool 30.

The free end 44 of the tool 30 has a generally rounded form with a central, slightly raised circular portion 60, as seen in FIG. 2, that provides a strike zone for receiving a blow from a tool, such as a hammer or mallet, during use of the tool 30. The central shaft 40 forms a handle by which the tool 30 may be grasped during use.

Referring to FIG. 2, the impactor head 50 includes a body 70 having a cavity 72 formed in an attachment end 74 of the impactor head 50. The cavity 72 includes cylindrical walls 76, 78, 80 that are concentrically disposed with respect to one another and stepped in diameter to form corresponding cylindrical cavities. In particular, the wall 76 has a diameter greater than diameters of the walls 78, 80 and the wall 78 has a diameter greater than the diameter of the wall 80. The walls 76, 78, 80 are sized to accommodate the cylinders 54, 56, 58, respectively, of the central projection 52 extending from the attachment end 42 of the tool 30, as will be discussed in greater detail below.

The impactor head 50 further includes an outer wall 81 having a cutout 82 formed therein, in which the cutout 82 is in partial communication with the cavity 72 and extends below the cavity 72. A protrusion 84 extends outwardly from a surface 86 formed in the cutout 82 and is spaced inwardly of the outer wall 81.

The impactor head 50 is illustratively formed from a polymeric material such as polyphenylsulphone as sold under the trade mark Radel R, but may be formed from other suitable materials.

The impactor head 50 is attached to the tool 30 by depressing the lever arm 46, thereby rotating the latch finger 48 about a hinge and lifting the latch finger 48. During this operation, the tool 30 is rotated until the latch finger 48 is aligned with the cutout 82 in the outer wall 80 of the impactor head 50 and the projection 52 of the tool 30 is inserted into the cavity 72 of the impactor head 50 with the cylinders 54, 56, 58 adjacent the walls 76, 78, 80, respectively. The projection 52 is inserted until an outer surface 90 of the cylinder 56 is adjacent an inner surface 92 forming a cylindrical cavity with the cylindrical wall 78. The lever arm 46 is thereafter released such that the latch finger 48 enters the cutout 82 and the latch 49 interferes with the protrusion 84 to prevent removal of the impactor head 50 from the tool 30. In a similar manner, the impactor head 50 may be removed by depressing the lever arm 46, thereby moving the latch finger 48 out of the cutout 82 and, thus, moving the latch 49 out of interference with the protrusion 84.

As discussed above, other instrument end pieces may be utilized with the tool 30. The other instrument end pieces may include structures similar to those of the impactor head 50, for example the cylindrical walls 76, 78, 80, the cutout 82, and the protrusion 84, to accommodate the attachment structures of the tool 30.

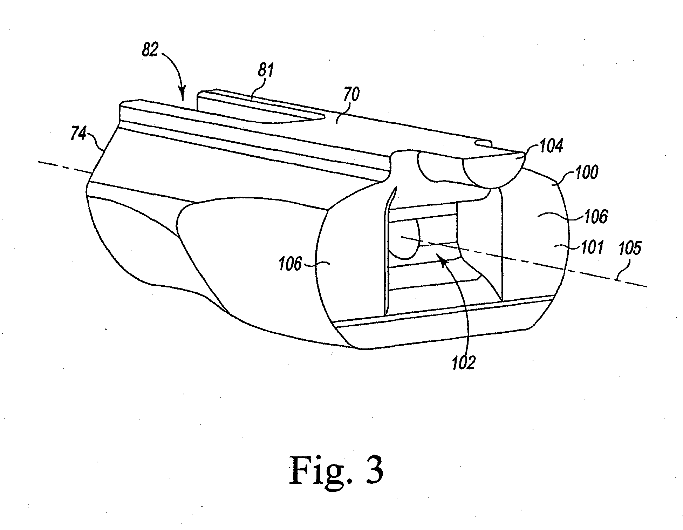

As best seen in FIG. 3, an impact end 100 of the impactor head 50 opposite the attachment end 74 includes a generally planar end wall 101 with a central cavity 102 formed in the planar end wall 101. In addition, an anti-rotation feature 104 extends outwardly and away from the planar end wall 101 adjacent the outer wall 81 of the impactor head 50. In particular, the anti-rotation feature 104 extends parallel to a longitudinal axis 105 of the impactor head 50.

The planar end wall 101 of the impactor head 50 includes impaction surfaces 106 disposed on opposing sides of the cavity 102, as will be discussed in greater detail below. While the end wall 101 is described as being planar, the end wall 101 may optionally have a different shape, for example, a shape that conforms to a shape of a tibial component 36 or other implant component with which the impactor head 50 is used.

As best seen in FIGS. 4 to 6, the tibial component 36 includes a platform 150 having an upper bearing surface 152 and a bottom surface 154. The tibial component 36 includes a stem 156 extending downwardly from the bottom surface 154 of the platform 150. The stem 156 is received into a bore 158 formed within the resected bone surface 34 of the tibia. The tibial component 36 is configured to be implanted within the bone surface 34, as will be discussed in detail below.

The upper bearing surface 152 of the tibial component 36 includes a medial bearing surface 160, a lateral bearing surface 162, and a spine 164 extending upwardly from the platform 150. The medial and lateral bearing surfaces 160, 162 are configured to receive or otherwise contact corresponding medial and lateral condyles of a femoral component (not shown). In this manner, the bearing surfaces 160, 162 may each have a concave contour. The spine 164 is positioned between the bearing surfaces 160, 162 and includes an anterior side 166 and a posterior side 168. The spine 164 is configured to be received within an intracondylar notch or recess formed between the medial and lateral condyles of the femoral component (not shown).

The tibial component 36 further includes an outer edge 180 with convex medial and lateral edges 182, 184, a convex anterior edge 186, and a concave posterior edge 188. The shape of the outer edge 180 may generally conform to a shape of an outer surface of the resected tibia 34.

Although specific steps during the surgical procedure will be described in detail, the tool 30 with the impactor head 50 attached may be used in any sequence of steps as preferred by a particular surgeon and depending on the steps necessary during the surgical procedure.

Use of the tool 30 with the attached impactor head 50 during a total knee replacement surgical procedure will now be briefly discussed with reference to FIGS. 4 to 6. After resection and reaming of the tibia, drilling of the proper holes in the patient's tibia 34, and appropriate sizing of the tibial components using trial components, the tool 30 and the impactor head 50 are used to secure the tibial component 36 to the patient's resected tibia 34. Bone cement may be used to help secure the tibial component 36 in place.

The impactor head 50 is attached to the tool 30 and the combination is used to secure the tibial component 36 within the resected tibia 34. In particular, once the tibial component 36 is positioned appropriately within the resected tibia 34, the impactor head 50 is moved toward the tibial component 36, as depicted in FIG. 4, such that the anti-rotation feature 104 of the impactor head 50 is aligned with the concave edge 188 of the tibial component 36 and the cavity 102 of the impactor head 50 is aligned with the spine 164.

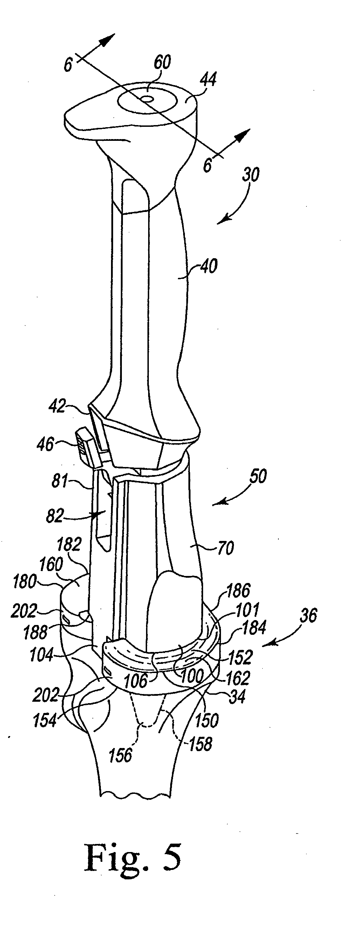

As depicted in FIG. 5, the impactor head 50 is positioned against the tibial component 36 with the impaction surfaces 106 of the impactor head 50 disposed adjacent the medial and lateral bearing surfaces 160, 162 of the tibial component 36. In this position, the cavity 102 allows the impaction surfaces 106 of the impactor head 50 to be disposed adjacent the bearing surfaces 160, 162 of the tibial component 36 by accommodating the spine 164 therein. In addition, when the anti-rotation feature 104 is positioned within a groove 200 formed by the concave edge 188, convex posterior edges 202 of the tibial component 36 prevent rotation of the impactor head 50.

After the impactor head 50 is positioned appropriately against the tibial component 36, the tibial component 36 is driven into the resected tibia 34 by using a hammer, mallet, or other suitable tool to strike the free end 44 of the tool 30. The force exerted by striking the free end 44 of the tool 30 drives the tibial component 36 into the bore 158 formed within the resected bone surface 34 of the tibia until the bottom surface 154 of the tibial component 36 is correctly seated against an upper resected surface 34 of the tibia. A surgeon may need to strike the free end 44 of the tool 30 multiple times to appropriately seat the tibial component 36.

The cavity 102 in combination with the spine 164 and/or the anti-rotation feature 104 in combination with the groove 200 allow for proper alignment of the impactor head 50 adjacent the tibial component 36 prior to and during use of the tool 30 and the impactor head 50. In this manner, proper alignment of the impactor head 50 with the tibial component 36 is facilitated and rotation of the tool 30 and the impactor head 50 is prevented. Forces transmitted by the tool 30 and the impactor head 50 will therefore be transmitted evenly and to the proper areas of the tibial component 36.

While a particular tibial implant component is depicted herein, instruments provided by the invention may be used with any tibial implant component. For example, the tibial component may form a portion of a cruciate retaining knee orthopaedic prosthesis in combination with a femoral component. Such a tibial component does not include a spine 164 and therefore, may be used with an impactor head 50 having the cavity 102 or may alternatively be used with an impactor head 50 that does not include the cavity 102. The tibial implant component may include a tibial tray and/or may include one or more pegs or other means for fixing the tibial implant component to a prepared tibial bone surface.

## Claims

1. An assembly for use in securing an orthopaedic implant within a surface of a patient's bone, comprising:
(a) an orthopaedic implant (36) which has a groove (200) formed in it, and
(b) an impactor (30) comprising:
a shaft (40) having a first end (44) adapted to be impacted by a tool, and
a head (50) having a first end (74) and a second end (100), in which the first end of the head is connected to the second end of the shaft and a second end of the head includes an impaction surface (106) and an anti-rotation projection (104) extending from the impaction surface and adapted for insertion within the groove formed in the orthopaedic implant to prevent rotation of the head and shaft when the head is in contact with the implant,
**characterised in that** the orthopaedic implant is an orthopaedic tibial implant and the groove (200) is provided by a concave portion of the posterior edge (188) of the orthopaedic tibial implant.

2. The assembly of claim 1, in which the projection (104) is parallel to a longitudinal axis (105) of the head (50) and extends from an edge of the impaction surface (106).

3. The assembly of claim 2, in which the implant (36) has an outwardly extending projection (164) and the head (50) has a cavity (102) formed in it in which the projection can be received when the head is in contact with the implant.

4. The assembly of claim 3, in which the head (50) has impaction surfaces (106) on opposing sides of the cavity (102) that are adapted to contact medial and lateral bearing surfaces (160, 162) or a perimeter of a surface of the tibial component.

5. The assembly of claim 3, in which the impaction surface (106) is formed on opposing sides of the cavity (102) and is generally planar.

6. The assembly of claim 3, in which the orthopaedic tibial implant (36) has concave bearing surfaces (160, 162), and in which the impaction surface (106) is formed on opposing sides of the cavity (106) and includes convex surfaces that are adapted to conform to the concave bearing surfaces of the implant.

7. The assembly of claim 2, in which the impactor head (50) is integrally and non-removably attached to the shaft (40).

8. The assembly of claim 2, in which the shaft (40) includes a connector by which the head (50) can be connected to the shaft (40).

9. The impactor of claim 8, in which the connector is formed by a lever arm (46) that operates a latch finger (48) having a latch (49) at an end thereof, and the head (50) has a projection (84) which cooperates with the latch to retain the head on the shaft (40).

## Patentansprüche

1. Eine Anordnung zur Verwendung bei einer Befestigung eines orthopädischen Implantats in einer Oberfläche eines Knochens eines Patienten, aufweisend:
(a) ein orthopädisches Implantat (36), dass eine darin ausgebildete Rille (200) aufweist, und
(b) einen Stoßkörper (30) aufweisend:
einen Schaft (40) mit einem ersten Ende (44), das angepasst ist, um durch ein Werkzeug einen Stoß zu erhalten, und
einen Kopf (50) mit einem ersten Ende (74) und einem zweiten Ende (100), wobei das erste Ende des Kopfes mit dem zweiten Ende des Schaftes verbunden ist und ein zweites Ende des Kopfes eine Stoßfläche (106) und einen Anti-Rotationsvorsprung (104) aufweist, der sich von der Stoßfläche erstreckt und für ein Einfügen in die im orthopädischen Implantat gebildete Rille angepasst ist, um eine Drehung des Kopfes und Schaftes zu verhindern, wenn sich der Kopf in Kontakt mit dem Implantat befindet,
**dadurch gekennzeichnet, dass** das orthopädische Implantat ein orthopädisches Tibiaimplantat ist und die Rille (200) durch einen konkaven Teil des posterioren Randes (180) des orthopädischen Tibiaimplantats bereitgestellt wird.

2. Anordnung nach Anspruch 1, bei der der Vorsprung (104) parallel zu einer Längsachse (105) des Kopfes 50 ist und sich von einem Rand der Stoßfläche (106) erstreckt.

3. Anordnung nach Anspruch 2, bei der das Implantat (36) einen sich auswärts erstreckenden Vorsprung (164) und der Kopf (50) eine Aussparung (102) aufweist, die darin gebildet ist, in der der Vorsprung aufgenommen werden kann, wenn sich der Kopf in Kontakt mit dem Implantat befindet.

4. Anordnung nach Anspruch 3, bei der der Kopf (50) Stoßflächen (106) auf gegenüberliegenden Seiten der Aussparung (102) aufweist, die angepasst sind, um mediale und laterale Lageroberflächen (160, 162) oder einen Perimeter einer Oberfläche der Tibiakomponente zu berühren.

5. Anordnung nach Anspruch 3, bei der die Stoßfläche (106) auf gegenüberliegenden Seiten der Aussparung 102 gebildet und im Allgemeinen eben ist.

6. Anordnung nach Anspruch 3, bei der das orthopädische Tibiaimplantat (36) konkave Lagerflächen (160, 162) aufweist, und bei der die Stoßfläche (106) auf gegenüberliegenden Seiten der Aussparung (106) ausgebildet ist und konvexe Oberflächen aufweist, die angepasst sind, so dass sie den konkaven Lageroberflächen des Implantats entsprechen.

7. Anordnung nach Anspruch 2, bei der der Stoßkörperkopf (50) integral und nicht abnehmbar am Schaft (40) befestigt ist.

8. Anordnung nach Anspruch 2, bei der der Schaft (40) ein Verbindungselement aufweist, durch das der Kopf (50) mit dem Schaft (40) verbunden sein kann.

9. Impaktor nach Anspruch 8, bei der das Verbindungselement durch einen Hebelarm (46) gebildet ist, der einen Sperrfinger (48) mit einer Klinke (49) an einem Ende desselben betätigt, und der Kopf (50) einen Vorsprung (48) aufweist, der mit der Klinke zusammenwirkt, um den Kopf auf dem Schaft (40) zu halten.

## Revendications

1. Ensemble destiné à être utilisé pour fixer un implant orthopédique à l'intérieur d'une surface de l'os d'un patient, comprenant :
(a) un implant orthopédique (36) qui a une rainure (200) formée dans ce dernier, et
(b) un impacteur (30) comprenant :
un arbre (40) ayant une première extrémité (44) adaptée pour être impactée par un outil, et
une tête (50) ayant une première extrémité (74) et une seconde extrémité (100), dans laquelle la première extrémité de la tête est raccordée à la seconde extrémité de l'arbre et une seconde extrémité de la tête comprend une surface d'impaction (106) et une saillie anti-rotation (104) s'étendant à partir de la surface d'impaction et adaptée pour être insérée à l'intérieur de la rainure formée dans l'implant orthopédique afin d'empêcher la rotation de la tête et de l'arbre lorsque la tête est en contact avec l'implant,
**caractérisé en ce que** l'implant orthopédique est un implant orthopédique tibial et la rainure (200) est fournie par une partie concave du bord postérieur (188) de l'implant orthopédique tibial.

2. Ensemble selon la revendication 1, dans lequel la saillie (104) est parallèle à un axe longitudinal (105) de la tête (50) et s'étend à partir d'un bord de la surface d'impaction (106).

3. Ensemble selon la revendication 2, dans lequel l'implant (36) à une saillie (164) s'étendant vers l'extérieur et la tête (50) a une cavité (102) formée dans cette dernière dans laquelle la saillie peut être reçue lorsque la tête est en contact avec l'implant.

4. Ensemble selon la revendication 3, dans lequel la tête (50) à des surfaces d'impaction (106) sur des côtés opposés de la cavité (102) qui sont adaptées pour entrer en contact avec des surfaces d'appui médiane et latérale (160, 162) ou un périmètre d'une surface du composant tibial.

5. Ensemble selon la revendication 3, dans lequel la surface d'impaction (106) est formée sur les côtés opposés de la cavité (102) et est généralement plane.

6. Ensemble selon la revendication 3, dans lequel l'implant orthopédique tibial (36) a des surfaces d'appui concaves (160, 162) et dans lequel la surface d'impaction (106) est formée sur des côtés opposés de la cavité (106) et comprend des surfaces convexes qui sont adaptées pour se conformer aux surfaces d'appui concaves de l'implant.

7. Ensemble selon la revendication 2, dans lequel la tête d'impacteur (50) est fixée de manière solidaire et non amovible à l'arbre (40).

8. Ensemble selon la revendication 2, dans lequel l'arbre (40) comprend un connecteur grâce auquel la tête (50) peut être raccordée à l'arbre (40).

9. Impacteur selon la revendication 8, dans lequel le connecteur est formé par un bras de levier (46) qui actionne un doigt de verrouillage (48) ayant un verrouillage (49) au niveau de son extrémité, et la tête (50) a une saillie (84) qui coopère avec le verrouillage afin de retenir la tête sur l'arbre (40).
